Europäisches Patentamt

European Patent Office

Office européen des brevets

(11) Publication number: **0 150 567 B1**

(12) **EUROPEAN PATENT SPECIFICATION**

(45) Date of publication of patent specification: **14.08.91**  (51) Int. Cl.⁵: **G01N 33/569**, G01N 33/531, G01N 33/546

(21) Application number: **84307188.7**

(22) Date of filing: **18.10.84**

(54) Process for detecting beta-hemolytic streptococcus antigens.

(30) Priority: **27.01.84 US 574461**

(43) Date of publication of application:
**07.08.85 Bulletin 85/32**

(45) Publication of the grant of the patent:
**14.08.91 Bulletin 91/33**

(84) Designated Contracting States:
**AT BE CH DE FR GB IT LI LU NL SE**

(56) References cited:

**CHEMICAL ABSTRACTS, vol. 93, no. 1, 7th July 1980, page 327, abstract no. 3369h, Columbus, Ohio, US; R. GUINET: "Rapid grouping of streptococci by latex agglutination after nitrous acid extraction", & ANN. BIOL. CLIN. (PARIS) 1980, 38(1), 15**

**BIOLOGICAL ABSTRACTS, vol. 75, no. 6, 1983, page 3953, abstract no. 38795, Philadelphia, Pa, US; R. GUINET et al.: "Serological grouping of streptococci: A collective evaluation in 6 laboratories of 3 rapid methods", & MED. MICROBIOL. IMMUNOL. 1982, 171(1), 23-32,**

**BIOLOGICAL ABSTRACTS, vol. 73, no. 12, 1982, page 8767, abstract no. 83982, Philadelphia, Pa, US; M. SLIFKIN et al.: "Serogrouping of beta-hemolytic strepococci from throat swabs with nitrous acid extraction and the Phadebact Streptococcus test", & J. CLIN. MICROBIOL. 1982, 15(1), 187-189 (Cat. D)**

(73) Proprietor: **MERIDIAN DIAGNOSTICS, INC.**
**3471 River Hills Drive**
**Cincinnati Ohio 45244(US)**

(72) Inventor: **Holland, Joseph Warren**
**9207 Maverick Drive**
**Cincinnati Ohio 45231(US)**
Inventor: **Walter, Richard Harold**
**9524 Heather Court**
**Blue Ash Ohio 45242(US)**

(74) Representative: **Myerscough, Philip Boyd et al**
**J.A.Kemp & Co. 14, South Square Gray's Inn**
**London, WC1R 5EU(GB)**

## Description

This invention relates to a rapid, simple process for detecting beta-hemolytic streptococcus antigen, particularly the Lancefield Group A antigen, in clinical specimens on a swab employing latex agglutination techniques. In particular the invention provides a process wherein antigens are extracted from the swab without resort to centrifugation.

Streptococci belonging to Lancefield beta-hemolytic Groups A, B, C, D, F and G are the ones most often associated with human streptococcal infection. Group A streptococci are the usual causative agent of pharyngitis and can lead to such long term problems as acute glomerulonephritis, rheumatic fever and impetigo. Group B streptococci are recognized as a serious neonatal pathogen, giving rise to purulent meningitis and pneumonia. Group C streptococci frequently cause infections similar to those of Group A as well as acute endocarditis. Group D and F streptococci are responsible for urogenital infection, endocarditis, nosocomial infections and thrombophlebitis. Group G streptococci comprise approximately 7% of all beta-hemolytic streptococcal isolates and have been reported as the cause of pharyngitis, wound infections, empyema and septicaemia.

Accurate diagnosis and treatment of streptococcal infections is possible only when the exact identity of the etiologic agent has been established. The high incidence of beta-hemolytic streptococcus infection in human beings has produced a need for an uncomplicated and sensitive procedure for the accurate serogrouping and typing of beta-hemolytic streptococci.

Various types of immuno-assays have been developed which are capable of detecting antigens found within the human body. These assays involve contacting the antigens in a test sample with antibodies which are specific for reaction to that type of antibody. In one such procedure, the antibody is bonded to the surface of minute particles and used to form an immune complex with the antigen in a test sample. Reaction of the antigens and antibodies causes agglutination of the antibody coated particles by forming an agglutination lattice which is easily discernible by the naked eye. In some cases, a suitable dye is used to improve the visibility of the agglutination lattice.

One well known agglutination technique of this type uses polystyrene latex particles. The latex particles are ordinarily less than one $\mu$m in diameter, preferably about 0.2 $\mu$m, and are negatively charged. The Fc portion of the antibody attaches to the surface of the latex particle by passive adsorption or covalent bonding. The Fab portion of the antibody which is not bonded to the latex particles extends outwardly and reacts directly with the group specific antigen, thus bonding the antigen to the antibody.

In order to make the antibody more sensitive to the antigen, the latex particles may be coated with a polypeptide containing carbohydrate units. Often the latex particles are sensitized with gamma globulin derived from rabbit, sheep or human blood, which may be altered to change its antigenic properties by treatment with an appropriate enzyme.

Immuno-assay procedures which use organic carrier particles have also been suggested. For example, GB-A-1,406,694 describes the use of killed staphylococcal bacteria coated with a polypeptide derived from Staphylococcus aureus or Staphylococcus epidermidis bacteria. This method of preparing antigen coated carrier particles for use in a coagglutination immuno-assay procedure has been used commercially in the Phadebact streptococcus test manufactured by Pharmacia Diagnostics, a division of Pharmacia, Inc.

Many streptococcal detection tests require extraction of the group specific carbohydrate antigens from the streptococcal cell to expose the antigens and increase the sensitivity of their reaction with the antibodies bonded to the coated carrier particles. Various carbohydrate extraction methodologies have been suggested by prior art workers.

For example, in the autoclave extraction method, a sample colony of beta-hemolytic streptococci or other bacterial species is subjected to a relatively high temperature, e.g. 121°C, in an autoclave. The sample is then centrifuged, and the supernatant fluid employed to serogroup one or more colonies using a latex agglutination procedure.

In the enzyme extraction procedure, the clinical sample is mixed with a suitable enzyme, e.g. S. albus filtrate reconstituted with an aqueous solution of lysozyme. The cell suspension is then incubated at elevated temperature and centrifuged to extract the supernatant. Test specimens for use with coagglutination reagents have also been prepared by the micronitrous acid extraction method described in "Serogrouping Single Colonies of Beta-Hemolytic Streptococci from Primary Throat Culture Plates with Nitrous Acid Extraction and Phadebact Streptococcal Reagents" by Slifkin et al., published in the Journal of Clinical Microbiology, Oct., 1980, at pp. 541, 545. In this procedure, colonies of Group A beta-hemolytic streptococci were prepared on a suitable growth medium. After a suitable period of incubation (24-48 hours) a sample of the lab culture was mixed with 20 microliters of 2M sodium nitrite solution. 3 microliters of glacial acetic acid were then added to form nitrous acid which serves to extract the carbohydrate antigen from the test sample. After a short incubation period, 16-24 mg of powdered sodium bicarbonate measured on a microspatula were added to the mixture to adjust the pH to 6.8 to 7.9. The solution was then brought to a final volume of about 80 microliters with distilled water. The mixture was then subjected to a coagglutination test such as the Phadebact coagglutination procedure referred to above. In "Serogrouping of Beta-Hemolytic Streptococci From Throat Swabs With Nitrous Acid Extraction and the Phadebact Streptococcus Test" by Slifkin et al, published in the Journal of Clinical Microbiology, Jan., 1982, at pp. 187-189, application of the nitrous acid extraction technique to the detection of Group A antigen of beta-hemolytic streptococci collected on throat swabs is described. In this procedure, the throat culture swab is placed in 0.3ml of distilled water and pressed against the inside of a vial to express fluid and bacteria from the swab into the vial.

The fluid is centrifuged for ten minutes and the supernatant decanted, with excess fluid in the vial removed by draining on filter paper. The pelleted cellular material remaining in the vial is mixed with 20 microliters of 4N sodium nitrite measured by means of a micropipette and 2.5 microliters of glacial acetic acid. 16-24 mg of powdered sodium bicarbonate is added by means of a microspatula, and a drop of the resulting extract mixed with the Phadebact coagglutination reagent. Previous micronitrous acid extraction methods have employed a pH indicator and 0.5N to 10N NaOH in place of the sodium bicarbonate to adjust the reaction mixture to pH 7.5 in order to permit an optimal coagglutination response.

The major drawbacks of known methods for extracting polysaccharide group antigens employed in agglutination test procedures is the length of time required to secure a result and the requirement of sophisticated laboratory equipment and reagents, as well as trained lab technicians to carry out the test procedures. For example, in some of the extraction procedures noted above, the test sample must be derived from a primary beta-hemolytic streptococcal colony, rather than directly from a throat swab sample. Furthermore, the micronitrous acid extraction technique employs extremely small quantities of reagents, on the order of several microliters. Accurately controlling the quantity and quality of such small amounts of the solutions is extremely difficult and requires a skilled technician, as well as precise measuring equipment such as micropipettes and microspatulas. Furthermore, the art teaches that a clinical test sample must be centrifuged before or during nitrous acid extraction. Centrifuge equipment is not only expensive to acquire and maintain, but must be operated by someone with the necessary training.

The present invention provides an in vitro diagnostic test procedure for the detection of beta-hemolytic streptococcal antigens by latex-agglutination assay. The procedure does not require sophisticated laboratory equipment and uses macroscopic quantities of liquid reagents, the measurement of which is non-critical. The test assay can be carried out in a few minutes directly on a collection swab in a physician's office by the physician, nurse or aide. Sterilization of the specimen is also achieved during the extraction procedure resulting in easy and safe disposal.

This invention provides a rapid means for detecting beta-hemolytic streptococcus antigens in clinical specimens by means of a latex agglutination technique. The procedure is effected without resort to centrifugation or other prior art extraction techniques requiring skilled laboratory personnel or expensive equipment.

According to the invention there is provided a process for detecting beta-hemolytic streptococcus antigens in a clinical specimen on a swab without centrifuging or culturing of the specimen comprising:

a) collecting a clinical specimen on a throat swab;
b) contacting the specimen on the swab with greater than 20 microlitres nitrous acid having a concentration of at least 75 micromoles/ml, at a temperature of from 20 to 28°C for from 1 minute to 1 hour, said nitrous acid having been formed by combining sodium nitrite solution and glacial acetic acid in the presence of the swab whereby the antigen is made more accessible by the nitrous acid solution;
c) thereafter adjusting the pH of the nitrous acid solution to 4.5 to 7.2 by means of a buffer;
d) removing a portion of the buffered acid solution;
e) mixing the removed buffered acid solution with minute latex particles coated with beta-hemolytic streptococcal antibodies; and
f) observing whether an agglutination lattice forms in the mixture, formation of said lattice indicating the presence of beta-hemolytic streptococcal antigens in the clinical specimen.

The invention thus provides an in vitro diagnostic test for the direct detection of Lancefield group A beta-hemolytic streptococcus antigens, particularly Lancefield group A beta-hemolytic streptococcus antigens in clinical specimens.

The procedure has specific application to the diagnosis of throat infections, particularly those caused by Group A beta-hemolytic streptococci. However, the test procedure may also be used for serogrouping antigens from other infected sites as well as Lancefield Group B, C, D, F and G antigens. The process of the invention is a presumptive screening technique. A positive test in the presence of supportive clinical signs of streptococcal pharyngitis enables early implementation of specific therapy. The procedure is easily followed by persons with limited technical skills yet provides the experienced microbiologist with a definitive serologic grouping.

In the practice of the invention a simple carbohydrate extraction procedure is performed directly on a swab containing a clinical sample suspected of containing the antigen in question. Detection is effected utilizing the principle of latex agglutination using immunoglobulin, specific for the particular streptococcus group in question, bound or passively adsorbed to uniform latex particles. No centrifuging is necessary to utilize the procedure of the invention. In addition macroscopic quantities of reagents are employed thereby eliminating the need for pipetting. As used herein macroscopic refers to quantities greater than about 20 microliters.

In the practice of the preferred embodiment of the present invention, a specimen is collected on a throat swab in the usual manner. The swab is then placed in a clean, dry microtube. Macroscopic quantities of nitrous acid are formed by mixing sodium nitrite and glacial acetic acid in the microtube. The acid serves to extract the carbohydrate antigen from the test sample on the swab. After a short period, a macroscopic quantity of a buffer solution, preferably a phosphate buffer, is added to the microtube to bring the mixture to a pH of 4.5-7.2.

At least a portion of the resulting solution is then removed from the microtube preferably by means of the swab and mixed with a latex agglutination detection reagent having particles coated with antibodies specific for the particular Lancefield group beta-hemolytic streptococci antigens. The detection reagent preferably comprises selected high titer rabbit gamma globulin passively adsorbed to latex spheres. The agglutination procedure is carried out on an appropriate glass slide or determination card. Formation of a visible agglutination lattice indicates the suspected antigen is present in the test sample.

More specifically the specimen collection swab used in the practice of the invention may be "Dacron" (Registered Trade Mark), cotton, rayon, or any commercially available fibrous transport swab, prepared in the usual way. In general the swab will be capable of absorbing about 0.3 ml. of liquid when saturated.

The carbohydrate extraction procedure of the present invention is as follows. The sample swab is removed from its transportation container and placed in a small container, such as a microtube, capable of holding the absorbent part of a saturated swab and about 100 microliters or more of additional liquid. One drop or about 42 microliters of 8M sodium nitrite solution are added to the microtube containing the swab. The sodium nitrite solution may be stored in and dispensed from a small plastic squeeze or dropper bottle or container. In a preferred embodiment, the dispensing bottle or container will dispense about 24 drops/ml., so that each drop contains about 42 microliters.

One drop (about 42 microliters) of a 2N glacial acetic acid solution is then added to the microtube. The glacial acetic acid solution may also be stored in and dispensed from a small plastic dropper or squeeze bottle or container of the type previously described.

The absorbent part of the swab is then rolled against the interior walls of the microtube so that liquid is expressed from the swab and reabsorbed and the sodium nitrite and acetic acid solutions are admixed and reacted to form nitrous acid which serves to extract the carbohydrate antigen from the test sample. In general, it is desirable to form sufficient nitrous acid to extract the maximum amount of antigen from the test sample. It has been found that a typical specimen collection swab will contain $10^4$-$10^8$ streptococcal organisms. Sufficient quantities of the sodium nitrite and acetic acid solutions (of whatever molarity) are used to produce at least 75 micromoles/ml of nitrous acid, provided the concentration of organisms is not reduced below the critical level of the detection reagent (i.e., about $10^5$ cells/ml.).

After formation of the nitrous acid the swab is allowed to remain in the tube at a temperature of 20 to 28°C for an incubation period of 1 minute to 1 hour, preferably about 5 minutes, to ensure that the carbohydrate extraction goes to completion. Two drops (about 84 microliters) of a 1M phosphate buffer (pH 7.2), preferably a combination of sodium phosphate monobasic and potassium phosphate dibasic, from a squeeze type dropper bottle of the type previously described are then added to the tube to produce a pH of the final solution of 4.5-7.2, preferably 6.8-7.2. In general, it is preferred that the buffer solution be as strong as possible, without precipitation of the buffer salts from solution. Other types of buffers may be used as well, particularly buffers formed from two or more salts in combination. The pH of the carbohydrate extracted sample is important to prevent chemical reaction with the group specific antibody during the agglutination procedure to be described hereinafter.

Following neutralization of the sample, the swab is rolled against the exterior surface of the tube walls so that liquid is expressed from the swab and reabsorbed. The swab optimally is allowed to rest briefly in the microtube for a period of a few seconds so that the maximum amount of liquid in the tube is absorbed by the swab. The extracted polysaccharide group antigen absorbed in the swab can thereafter be used with an appropriate agglutination serogrouping technique for the specific group antigen.

For example, the swab may be removed from the microtube and gently rolled on the surface of a glass slide to express a portion of the sample liquid. One or two drops of a detection reagent is then added to the solution on the slide, and the slide rocked gently back and forth to mix the sample solution and the detection reagent. If the test is positive, formation of an agglutination lattice should be observed in about 1-5 minutes, usually about 2 minutes. Presence of the lattice indicates the presence of the group specific antigen in the original throat sample.

The detection reagent used in the practice of the present invention preferably comprises high titer immune rabbit gamma globulin specific to the suspected antigen passively adsorbed to 0.8 $\mu$m latex spheres (i.e. latex spheres in the range of 0.790 to 0.810 $\mu$m). The rabbit gamma globulin used in connection with the detection reagent of the present invention is "selected", that is the globulin serum, when diluted for optimal activity, must result in a detection reagent capable of formation of an agglutination lattice in the presence of a test sample having at least $10^5$ streptococci cells/ml. of solution. The latex particles may also be buffered in a phosphate buffer solution containing 0.01% thimerosal as a preservative, if desired.

It should be noted that the procedure of the present invention is a screening test and will not distinguish between colonized individuals and those harboring an infection. Furthermore, the procedure will show a positive reaction only for antigens specific to the antibodies coating the latex particles of the detection reagent.

In a modification of the preferred embodiment of the present invention, positive and negative control reagents are provided. The positive control reagent is formed from a killed suspension of beta-hemolytic streptococcal cells, e.g. Group A equivalent in activity to an extracted solution of $5 \times 10^6$ per cm³. The negative control reagent comprises 0.8 $\mu$m latex particles to which normal, i.e. unimmunized, gamma globulin is adsorbed. The serum used should be gamma globulin, screened for lack of cross reactivity and false positives.

In this embodiment of the invention, the carbohydrate extracted solution is prepared as described hereinabove. The swab with the absorbed solution is removed from the microtube and rolled briefly over the surface of three spaced areas on a glass determination slide. Alternatively, a slide or card made from a synthetic paper such as KIMDURA manufactured by Kimberly Clark having the three designated areas may be used. It has been found that the use of this particular paper prevents spontaneous agglutination of the detection reagent. One of the areas is for the negative control, one area is for the specimen, and one area is for the positive control.

Two drops of the positive control reagent, which may be stored in and dispensed from a plastic squeeze or dropper bottle of the type described above is added to the positive area. One drop of the negative control reagent which may be stored in and dispensed from a similar type of plastic squeeze or dropper bottle is added to the negative area on the slide. One drop of the detection reagent, which may be stored in and dispensed from a similar type of plastic squeeze or dropper bottle is added to the specimen area of the determination card or slide.

The card or slide is then rocked about its long axis, optimally approximately 45° in each direction at the rate of about 30 cycles per minute under a bright light source. As the slide is rocked, each of the marked areas is carefully observed for signs of formation of the agglutination lattice.

In some instances, the specimen may cause a small amount of agglutination to occur in both the negative and specimen areas. While this is permissible, the agglutination in the specimen area must be stronger than that in the negative area for the test to be valid. If more agglutination occurs in the negative control, the test is invalid, since some abnormality in the specimen has caused a non-specific, i.e. invalid, test result. In this case, a culture must be performed to determine the patient's status.

If no agglutination lattice is observed in the positive control, the reagents used are ineffective and should be discarded. The test should be repeated with active reagents.

If more agglutination occurs in the specimen area than in the negative area, the test is considered positive. In this instance, a culture should be necessary only for epidemiology, or if treatment appears to be unsuccessful.

In yet another embodiment of the invention, a plurality of detection reagents are used, each specific to a particular antigen. For example, five separate dispenser containers or bottles may be provided, each containing a detection reagent composed of latex particles coated with antibodies specific to the Groups A, B, C, D, F or G beta-hemolytic streptococci antigen. That is, the Group A detection reagent will form an agglutination lattice only with Group A antigens, the Group B detection reagent will form an agglutination lattice only with Group B antigens, and so forth.

The determination card or slide may also be modified to include areas designated for each of the group specific detection reagents. The carbohydrate extracted sample on the swab may be separated into individual portions by means of a micropipette, and one of the separate samples applied to each of the designated areas on the determination card. One drop of each of the detection reagents is then added to the associated designated area, and a visual inspection made for formation of the agglutination lattice in one of the designated areas. It will be observed that in this test, the other groups act as negative controls for the group being tested. However, in some instances, the presence of more than one group specific antigen in the test sample may cause a positive reaction in more than one of the designated areas. In this situation, further testing for the specific group antigen is necessary.

Using the procedure of the present invention, in clinical trials for the detection of Group A beta-hemolytic streptococci on 161 patient specimens, no specimens (27/27) containing beta-hemolytic streptococci other than Group A were found to react in the test. Three specimens found to be negative when cultured, but positive using the present invention were later confirmed as culturally positive in small numbers on follow-up testing. These date indicate a specificity of 100%. All specimens found to be positive for Group A beta-hemolytic streptococci by the culture method were found to be positive by the method of the present invention. In addition, three specimens found negative on primary culture but positive by the present invention were later confirmed as positive in small numbers. Thus, the sensitivity of the method of the present invention is at least that of culture methods.

It will be observed that the test reagents and apparatus used with the present invention may be provided as part of a test kit. For example, the kit may contain separate small plastic squeeze or dropper bottles or containers containing each of the three extraction reagents, and the detection reagent, alone or with the positive control and negative control reagents. The kit may further include a supply of microtubes and/or determination cards or slides. Kits may also be used which include containers of the three extraction reagents, a plurality of detection reagents, each specific for a different antigen, micropipettes, microtubes and determination cards or slide, as previously described. The kits, which supply sufficient materials for a number of tests, may be made available to private physicians, clinics, hospital emergency rooms and the like. As noted hereinabove, the test procedure and kit of the present invention make it possible for diagnosis of the presence of beta-hemolytic streptococcus to be made within minutes of taking the specimen with immediate institution of treatment and elimination of the delays and inefficiencies associated with culture methods or methods which require clinical laboratory equipment and personnel.

It will be understood that various changes in the details, steps, materials and arrangements of parts, which have been herein described and illustrated in connection with the present invention, may be made. For example, liquid buffer solutions made from borates, other phosphates, other salts in combination or other strong buffer may also be used. Other concentrations of the extraction reagents may also be utilized, provided the concentration ranges noted for the resulting nitrous acid solution are maintained. Furthermore, it will be understood that the present invention may be used to detect the presence of other organisms which produce carbohydrate antigens, e.g. fungus and bacteria. Likewise, a suitable dye may be used with the latex agglutination detection reagent to enhance the visibility of the agglutination lattice.

It will also be understood that as used herein, antibody means any substance in the blood serum or other fluid of the body which exerts a specific restrictive or destructive action on bacteria or other noxa or neutralizes their toxin. Furthermore, antigen means any substance which, when introduced into an animal organism, including humans, causes the production of an antibody.

From the foregoing description, it is apparent that the present invention does not require centrifuging or the accurate mixing of very minute amounts of reagents in the detection procedure. Furthermore, all of the test reagents are in liquid form and may be dispensed drop-by-drop. Consequently, the test procedure lends itself to use by physicians, nurses or aides who are unskilled in complex laboratory procedures.

## Claims

1. A process for detecting beta-hemolytic streptococcus antigens in a clinical specimen on a swab without centrifuging or culturing of the specimen comprising:

   a) collecting a clinical specimen on a throat swab;

   b) contacting the specimen on the swab with greater than 20 microlitres nitrous acid having a concentration of at least 75 micromoles/ml, at a temperature of from 20 to 28°C for from 1 minute to 1 hour, said nitrous acid having been formed by combining sodium nitrite solution and glacial acetic acid in the presence of the swab whereby the antigen is made more accessible by the nitrous acid solution;

c) thereafter adjusting the pH of the nitrous acid solution to 4.5 to 7.2 by means of a buffer;

d) removing a portion of the buffered acid solution;

e) mixing the removed buffered acid solution with minute latex particles coated with beta-hemolytic streptococcal antibodies; and

f) observing whether an agglutination lattice forms in the mixture, formation of said lattice indicating the presence of beta-hemolytic streptococcal antigens in the clinical specimen.

2. A process according to claim 1 wherein the sodium nitrite solution is an 8M solution.

3. A process according to claim 1 or 2 wherein the glacial acetic acid solution is a 2N solution.

4. A process according to claim 1 wherein about 42 microliters of 8M sodium nitrite solution and about 42 microliters of 2N glacial acetic acid are combined.

5. A process according to any one of the preceding claims wherein said buffer comprises a phosphate buffer solution.

6. A process according to claim 5 wherein said buffer solution comprises a 1M phosphate buffer solution having a pH of 7.2.

7. A process according to claim 6 wherein the buffer comprises about 82 microliters of a 1M phosphate buffer solution.

8. A process according to any one of the preceding claims wherein the antibody coating comprises high titer rabbit gamma globulin passively adsorbed on latex spheres, said gamma globulin when diluted for optimal activity being able to detect at least $5 \times 10^5$ streptococcal cells/ml of solution.

9. A process according to claim 8 wherein said latex spheres are 0.8 $\mu$m in diameter.

10. A process according to any one of the preceding claims wherein the buffered acid solution is removed by means of the swab.

11. A process according to any one of the preceding claims wherein the specimen on the swab is contacted with the nitrous acid for about 5 minutes.

12. A process according to any one of the preceding claims wherein the swab contains from $10^4$ to $10^8$ beta-hemolytic streptococcus antigens.

13. A test kit suitable for use in a process as defined in any one of the preceding claims which comprises containers containing sodium nitrite solution, glacial acetic acid and a buffer and minute latex particles coated with beta-hemolytic streptococcal antibodies.

**Revendications**

1. Procédé de détection d'antigènes streptococcaux bêta-hémolytiques dans un échantillon clinique sur un tampon d'ouate sans centrifugation ou culture de l'échantillon, dans lequel:

a) on recueille un échantillon clinique dans la gorge avec un tampon d'ouate;

b) on met en contact l'échantillon sur le tampon d'ouate avec plus de 20 microlitres d'acide nitreux ayant une concentration d'au moins 75 micromoles/ml, à une température allant de 20 à 28°C pendant de 1 minute à 1 heure, ledit acide nitreux ayant été formé en combinant une solution de nitrite de sodium et d'acide acétique glacial en présence du tampon, ce qui fait que l'antigène est rendu plus accessible par la solution d'acide nitreux;

c) puis on ajuste le pH de la solution d'acide nitreux entre 4,5 et 7,2 au moyen d'un agent tampon ;

d) on enlève une partie de la solution d'acide tamponnée;

e) on mélange la solution d'acide tamponnée enlevée avec de très petites particules de latex revêtues d'anticorps streptococcaux bêta-hémolytiques; et

f) on observe s'il se forme un réseau d'agglutination dans le mélange, la formation dudit réseau indiquant la présence d'antigènes streptococcaux bêta-hémolytiques dans l'échantillon clinique.

2. Procédé selon la revendication 1 dans lequel la solution de nitrite de sodium est une solution 8 M.

3. Procédé selon la revendication 1 ou 2 dans lequel la solution d'acide acétique glacial est une solution 2 N.

4. Procédé selon la revendication 1 dans lequel on combine environ 42 microlitres de solution 8 M de nitrite de sodium et environ 42 microlitres d'acide acétique glacial 2 N.

5. Procédé selon l'une quelconque des revendications précédentes dans lequel ledit tampon de pH comprend une solution de tampon phosphaté.

6. Procédé selon la revendication 5 dans lequel ladite solution tampon comprend une solution de tampon phosphaté 1 M ayant un pH de 7,2.

7. Procédé selon la revendication 6 dans lequel le tampon comprend environ 82 microlitres d'une solution de tampon phosphaté 1 M.

8. Procédé selon l'une quelconque des revendications précédentes dans lequel le revêtement d'anticorps comprend de la gamma-globuline de lapin de titre élevé passivement adsorbée sur des sphères de latex, ladite gamma-globuline lorsqu'elle est diluée en vue d'une activité optimale étant capable de détecter au moins $5 \times 10^5$ cellules streptococcales/ml de solution.

9. Procédé selon la revendication 8 dans lequel lesdites sphères de latex ont un diamètre de 0,8 $\mu$m.

10. Procédé selon l'une quelconque des revendications précédentes dans lequel la solution d'acide tamponnée est enlevée au moyen du tampon d'ouate.

11. Procédé selon l'une quelconque des revendications précédentes dans lequel l'échantillon se trouvant sur le tampon d'ouate est mis en contact avec l'acide nitreux pendant environ 5 minutes.

12. Procédé selon l'une quelconque des revendications précédentes dans lequel le tampon d'ouate contient de $10^4$ à $10^8$ antigènes streptococcaux bêta-hémolytiques.

13. Ensemble pour test approprié pour une utilisation selon le procédé tel que défini selon l'une quelconque des revendications précédentes qui comprend des récipients contenant une solution de nitrite de sodium, de l'acide acétique glacial et un agent tampon et de très petites particules de latex revêtues d'anticorps streptococcaux bêta-hémolytiques.

**Patentansprüche**

1. Verfahren zum Nachweis von beta-hämolytischen Streptokokken-Antigenen in einer klinischen Probe auf einem Abstrich ohne Zentrifugierung oder Kultivierung der Probe umfassend:

a. Sammeln einer klinischen Probe an einem Rachenabstrich;

b. Inkontaktbringen der Probe auf dem Abstrich mit mehr als 20 $\mu$l salpetriger Säure mit einer Konzentration von mindestens 75 $\mu$Mol/ml, bei einer Temperatur von 20 bis 28°C in einem Zeitraum von einer Minute bis einer Stunde, wobei die salpetrige Säure durch Kombination von Natriumnitritlösung und Eisessig in Gegenwart des Abstriches gebildet wurde, wodurch das Antigen für die Lösung der salpetrigen Säure zugänglicher gemacht wird;

c. danach Einstellen des pH-Wertes der Lösung der salpetrigen Säure auf 4.5 bis 7.2 mittels eines Puffers;

d. Abtrennen eines Teils der gepufferten Säurelösung;

e. Mischen der abgetrennten gepufferten Säurelösung mit sehr kleinen Latexteilchen, die mit beta-hämolytischen Streptokokken-Antikörpern beschichtet sind; und

f. Beobachten, ob sich in der Mischung ein Agglutinations-Gitter ausbildet, wobei die Ausbildung eines solchen Gitters die Gegenwart von beta-hämolytischen Streptokokken-Antigenen in der klinischen Probe anzeigt.

2. Verfahren nach Anspruch 1, dadurch gekennzeichnet, daß die Natriumnitritlösung eine 8M-Lösung ist.

3. Verfahren nach Anspruch 1 oder 2, dadurch gekennzeichnet, daß die Eisessig-Lösung eine 2N-Lösung ist.

4. Verfahren nach Anspruch 1, dadurch gekennzeichnet, daß ca. 42 $\mu$l 8M Natriumnitritlösung und ca. 42 $\mu$l 2N Eisessiglösung vereinigt werden.

5. Verfahren nach einem der vorhergehenden Ansprüche, dadurch gekennzeichnet, daß der Puffer eine Phosphatpufferlösung umfaßt.

6. Verfahren nach Anspruch 5, dadurch gekennzeichnet, daß die Pufferlösung eine 1M-Phosphatpufferlösung mit einem pH-Wert von 7.2 umfaßt.

7. Verfahren nach Anspruch 6, dadurch gekennzeichnet, daß der Puffer ca. 82 $\mu$l einer 1M-Phosphatpufferlösung umfaßt.

8. Verfahren nach einem der vorhergehenden Ansprüche, dadurch gekennzeichnet, daß die Antikörperbeschichtung Kaninchen-gamma-Globulin von hohem Titer umfaßt, das an Latexkügelchen passiv adsorbiert ist, und das gamma-Globulin, wenn es für optimale Aktivität verdünnt ist, dazu fähig ist, mindestens 5 x 10$^5$ Streptokokken-Zellen/ml Lösung nachzuweisen.

9. Verfahren nach Anspruch 8, dadurch gekennzeichnet, daß die Latexkügelchen einen Durch-messer von 0.8 $\mu$m besitzen.

10. Verfahren nach einem der vorhergehenden Ansprüche, dadurch gekennzeichnet, daß die gepufferte Säurelösung mittels des Abstriches abgetrennt wird.

11. Verfahren nach einem der vorhergehenden Ansprüche, dadurch gekennzeichnet, daß die Probe auf dem Abstrich mit der salpetrigen Säure ca. 5 Minuten kontaktiert wird.

12. Verfahren nach einem der vorhergehenden Ansprüche, dadurch gekennzeichnet, daß der Ab-strich 10$^4$ bis 10$^8$ beta-hämolytische Streptokokken-Antigene enthält.

13. Testkit zur Verwendung in einem gemäß ei-nem der vorhergehenden Ansprüche definier-ten Verfahren, das Behälter umfaßt, die Natriumnitritlösung, Eisessig und einen Puffer enthalten, und sehr kleine Latexteilchen, die mit beta-hämolytischen Streptokokken-Antikörp-ernbeschichtet sind.